# EUROPÄISCHE PATENTSCHRIFT

(11) **EP 2 717 937 B1**
(45) Veröffentlichungstag und Bekanntmachung des Hinweises auf die Patenterteilung: **25.05.2016**
(21) Anmeldenummer: 12726444.8
(22) Anmeldetag: 08.06.2012
(51) Int. Cl.: A61M 1/10, A61M 1/16, A61M 1/26, A61M 1/34

(54) **PORTABLE DIALYSEVORRICHTUNG**
PORTABLE DIALYSIS DEVICE
DISPOSITIF DE DIALYSE PORTABLE

(30) Priorität: 10.06.2011 DE 102011103932
(43) Veröffentlichungstag der Anmeldung: 16.04.2014
(73) Patentinhaber: Dualis Medtech GmbH, 82229 Seefeld (DE)
(72) Erfinder: SCHILLER, Wolfgang, 53125 Bonn (DE); SCHMID, Thomas, 82211 Herrsching (DE)
(74) Vertreter: von Kreisler Selting Werner - Partnerschaft von Patentanwälten und Rechtsanwälten mbB
(86) Internationale Anmeldenummer: PCT/EP2012/060867
(87) Internationale Veröffentlichungsnummer: WO 2012/168412

(56) Entgegenhaltungen:
- DE-A1- 19 821 543
- FR-A1- 2 276 856
- US-A1- 2006 030 809
- US-A1- 2006 253 194
- US-B1- 6 607 368

## Beschreibung

Die Erfindung betrifft eine portable Dialysevorrichtung.

Weltweit existiert eine sehr große Anzahl an Patienten, die nierenkrank sind und eine Nierenersatztherapie benötigen. Aktuell findet der wesentlichste Teil der Nierenersatztherapie in Form von Dialysebehandlungen statt. Hierbei findet ein Stoffaustausch über eine Membran statt, wobei sich auf der einen Seite der Membran Blut oder Plasma und auf der anderen Seite der Membran ein Dialysat befindet.

Die Dialyse findet einerseits durch Diffusion statt. Diffusion ist ein Partikelaustausch von derjenigen Seite der Membran, die die höhere Konzentration an Partikeln aufweist, zu der Seite mit der niedrigeren Konzentration. Zum Durchführen der Diffusion muss somit ein Konzentrationsgradient bestehen.

Weiterhin nutzt die Dialyse das Prinzip der Ultrafiltration, bei dem mittels eines Druckgradienten Flüssigkeit und/oder Teilchen durch die Membran hindurchtreten.

Zusätzlich zu den genannten Aspekten können auch Osmoseeffekte bei der Dialyse genutzt werden.

Derzeit bekannte Dialyseverfahren benötigen sehr große Membranoberflächen, da ein Blutreinigungseffekt in relativ kurzer Zeit (z.B. dreimal wöchentlich je 4 bis 6 Stunden) erreicht werden soll. Bisher bekannte Dialysegeräte begegnen diesem Problem durch Verwendung einer Vielzahl von Membranröhren mit einem sehr kleinen Durchmesser, durch die das Blut geleitet wird. Trotz dieser großen Membranflächen ist es für den Patienten notwendig, die genannten Dialysezeiten mehrmals wöchentlich einzuhalten. Ein weiteres Problem bei aus dem Stand der Technik bekannten Dialyseverfahren ist, dass ein großer Gefäßzugang benötigt wird, der den erforderlichen großen Volumenstrom gewährleisten kann. Hierfür werden bspw. arterio-venöse Shunts verlegt, die einen "Kurzschluss" zwischen einer Arterie und einer Vene des Patienten darstellen. Derartige Shunts stellen eine hohe Kreislaufbelastung für den Patienten dar.

Aufgabe der Erfindung ist es eine Vorrichtung und ein Verfahren zu schaffen, die eine komfortable Dialyse ermöglichen.

Die Lösung der Aufgabe erfolgt erfindungsgemäß durch die Merkmale des Anspruchs 1.

Die erfindungsgemäße portable Dialysevorrichtung ist kontinuierlich im oder am Körper eines Patienten tragbar. Sie weist eine Blutkammer auf, in die Blut des Patienten aufnehmbar ist. Ferner weist sie eine mit einem Hydraulikfluid befüllbare Hydraulikkammer auf, die an die Blutkammer angrenzt. Zwischen der Hydraulikkammer und der Blutkammer ist eine wenigstens teilweise flexible Fördermembran angeordnet. Flexibel bedeutet in diesem Zusammenhang, dass die Fördermembran in ihrer Form derart veränderlich ist, dass zumindest ein Teil von ihr in Richtung der Blutkammer oder in Richtung der Hydraulikkammer verschiebbar ist. Durch die Fördermembran werden somit die Blutkammer und die Hydraulikkammer voneinander getrennt. Die Fördermembran ist während des Vorgangs der Befüllung der Hydraulikkammer mit Hydraulikfluid derart in Richtung der Blutkammer bewegbar, dass ein Komprimieren der Blutkammer und somit ein Auswurf des hierin befindlichen Blutes erfolgt.

Die Dialysevorrichtung weist ferner eine Pumpe zum steuerbaren Befüllen und/oder Entleeren des Hydraulikfluids in der Hydraulikkammer auf, sodass hierdurch Blut in die Blutkammer und/oder aus dieser hinaus förderbar ist.

Ferner ist zwischen der Blutkammer und der Hydraulikkammer eine Filtermembran angeordnet, durch die im Blut befindliche Abfallstoffe an das Hydraulikfluid in der Hydraulikkammer abführbar sind, sodass das Hydraulikfluid gleichzeitig als Dialysat dient.

Durch die erfindungsgemäße portable Dialysevorrichtung ist eine kontinuierliche Dialyse im oder am Körper des Patienten rund um die Uhr möglich. Aufgrund dieser kontinuierlichen Dialyse kann die erforderliche Membranfläche gegenüber bisher bekannten Dialysevorrichtungen erheblich reduziert werden. Bspw. kann die erfindungsgemäße Dialysevorrichtung eine Filtermembran mit einer Membranfläche von 2 cm² bis 200 cm², insbesondere 5 cm² bis 50 cm² aufweisen. Wesentlich bei der Durchführung einer kontinuierlichen Dialyse rund um die Uhr ist ein kontinuierlicher Austausch des zu reinigenden Blutes. Erfindungsgemäß wird hierfür eine Pumpe verwendet. Jedoch wird durch die Pumpe das Blut des Patienten nicht unmittelbar gefördert, da dies aufgrund der mechanischen Belastung beim Pumpen zu einer Beschädigung der Substanzen im Blut führen würde. Stattdessen ist eine komprimierbare Blutkammer vorgesehen, die durch eine flexible Membran komprimiert wird. Die Membran wird in Richtung der Blutkammer bewegt, indem die an die Blutkammer angrenzende Hydraulikkammer mit einem Hydraulikfluid, das durch die Pumpe gefördert wird, befüllt wird. Durch ein entsprechendes Ansteuern der Pumpe ist es somit möglich, die Fördergeschwindigkeit, die Förderfrequenz und den Förderdruck zu steuern mit dem Blut in die Blutkammer und aus dieser hinaus gefördert wird. Es ist somit eine exakte Steuerung des Dialysevorgangs möglich.

Wesentliches Merkmal der erfindungsgemäßen portablen Dialysevorrichtung ist ferner die Verwendung des Hydraulikfluids, das die Kompression der Blutkammer verursacht, als Dialysat. Anders ausgedrückt wird als Hydraulikfluid eine Flüssigkeit verwendet, die in bisher bekannten Dialysevorrichtungen als Dialysat verwendet wird. Abfallstoffe, die im Rahmen der Dialyse aus dem Blut entfernt werden sollen, können somit aus dem in der Blutkammer befindlichen Blut durch die Filtermembran in das in der Hydraulikkammer befindliche Dialysat übertreten. Dies kann durch Diffusions- oder Osmoseprozesse über einen Konzentrationsgradienten und/oder durch eine Ultrafiltration über einen Druckgradienten geschehen. Besonders vorteilhaft ist bei der Ultrafiltration, dass durch die Verwendung der Pumpe in der erfindungsgemäßen Dialysevorrichtung der Druckgradient zwischen der Druckkammer und der Dialysekammer präzise einstellbar ist.

Durch die Erfindung ist es somit gelungen, eine sehr kompakte Dialysevorrichtung zu schaffen, die permanent im oder am Körper des Patienten getragen werden kann, sodass eine permanente Dialyse durchgeführt werden kann. Dies wiederrum erlaubt eine signifikante Verringerung der erforderlichen Membranfläche für die Dialyse. Zeitaufwendige Besuche in einem Dialysezentrum, wie sie bisher notwendig waren, können somit entfallen. Weiterhin kann auf das Verlegen eines Shunts, der bei bisher bekannten Dialysevorrichtungen notwendig ist, verzichtet werden. Die Belastung für das Kreislaufsystem des Patienten wird somit erheblich verringert.

Um zu verhindern, dass sich das gerade gereinigte Blut aus der Blutkammer mit frischem, noch nicht gereinigtem Blut vermischt, ist es möglich, einen getrennten Zu- und Abfluss zur Blutkammer mit einem jeweils separaten Ventil zu verwenden. Beim Befüllen der Blutkammer kann das Zufuhrventil des Zuflusses geöffnet werden, sodass die Blutkammer mit frischem noch nicht gereinigtem Blut gefüllt wird, während dieses Ventil verschlossen wird, um das Blut aus der Blutkammer auszuwerfen, nachdem es gereinigt wurde. Hierfür wird das Auslassventil des Auslasses geöffnet.

Alternativ ist es möglich ein gemeinsamen Zu- und Abfluss zur Blutkammer vorzusehen, der möglichst nah an dem Blutgefäß angeordnet ist, mit dem die Dialysevorrichtung verbunden ist. Durch den ständigen Blutfluß im Blutgefäß wird das gereinigte Blut, das von der Blutkammer ausgeworfen wird, weitergefördert, sodass beim nächsten Befüllen der Blutkammer diese zum größten Teil mit frischem noch nicht gereinigten Blut aus dem Blutgefäß gefüllt wird. Wichtig ist hierbei, dass das Totvolumen, d.h. dasjenige Blutvolumen in der Zuführleitung vom Blutgefäß zu der Blutkammer möglichst klein ist, da hier keine Blutzirkulation stattfindet.

Es ist bevorzugt, dass neben der Verbindung über die Filtermembran keine weitere Verbindung zwischen der Blutkammer und der Hydraulikkammer besteht, über die ein insbesondere unmittelbarer Flüssigkeits- oder Teilchenaustausch möglich wäre. Zum Aufnehmen von Blut kann die Blutkammer mittels aus dem Stand der Technik bekannter Adapter an das Kreislaufsystem des Patienten, bspw. an die Aorta oder mit dem venösen System verbunden werden. Hierfür können handelsübliche Gefäßprothesen verwendet werden.

In einer bevorzugten Ausführungsform sind die Filtermembran und die Fördermembran dasselbe Bauteil, sodass durch die Förder- und Filtermembran gleichzeitig ein Komprimieren der Blutkammer und ein Filtern des Blutes erfolgt. Die Fördermembran kann hierzu bspw. Poren aufweisen, durch die Abfallstoffe, die vom Blut entfernt werden sollen, von der Blutkammer in die Hydraulikkammer übertreten können. Unter den Abfallstoffen, die aus dem Blut entfernt werden sollen, werden erfindungsgemäß diejenigen Stoffe verstanden, die im Rahmen einer aus dem Stand der Technik bekannten Dialyse ebenfalls aus dem Blut entfernt werden, bspw. harnpflichtige Substanzen, Nephrotoxine und Elektrolyte. Die Filtermembran weist eine derartige Porengröße auf, dass die genannten Substanzen durchgelassen werden aber größere Moleküle, wie bspw. Eiweiße und Blutzellen zurückgehalten werden. Ferner ist es durch die erfindungsgemäße Dialysevorrichtung möglich, dem Blut des Patienten durch Anlegen eines Druckgradienten (d.h. durch Erzeugen eines Unterdrucks auf der Dialysatseite) Flüssigkeit zu entziehen. Durch Umkehrung dieses Druckgradienten kann erfindungsgemäß ferner diese Flüssigkeit dem Patienten wieder zugeführt werden.

In einer alternativen Ausführungsform sind die Filtermembran und die Fördermembran als separate Bauteile ausgebildet. In dieser Ausführungsform kann die Filtermembran bspw. unflexibel sein, da sie nicht zur Komprimierung der Blutkammer verwendet wird.

Es ist weiterhin bevorzugt, dass die Drehrichtung der Pumpe umkehrbar ist. Ferner ist bevorzugt, dass der durch die Pumpe geförderte Volumenstrom des Hydraulikstroms regelbar ist. Hierdurch kann durch die Pumpe oder mit Unterstützung der Pumpe Blut in einem definierten Volumenstrom in die Blutkammer und aus dieser hinaus gefördert werden.

Durch die Regelung des durch die Pumpe geförderten Volumenstroms des Hydraulikfluids ist somit der Druckgradient zwischen dem Blut und dem als Dialysat dienenden Hydraulikfluid zur Regelung der Blutreinigung durch Ultrafiltration veränderbar, indem in der Hydraulikkammer ein höherer oder niedrigerer Druck als in der Blutkammer einstellbar ist.

Es ist weiterhin bevorzugt, dass durch die Pumpe ein kontinuierliches Fördern von Blut ohne Unterbrechung in die Blutkammer und aus dieser hinaus erfolgt, wobei insbesondere der Volumenstrom des in die Blutkammer geförderten Bluts bei 0,4 l bis 2,5 l, insbesondere bei 0,8 l bis 1,5 l pro Minute bei erwachsenen Patienten und bei 50 ml bis 800 ml, insbesondere 100 ml bis 400 ml pro Minute bei Kindern liegt.

Es ist weiterhin bevorzugt, dass die Blutkammer und die Hydraulikkammer in einem inkompressiblen Gehäuse angeordnet sind. Ferner kann die Blutkammer eingangsseitig einen inkompressiblen Abschnitt aufweisen, an dessen bzgl. der Bluteinlassströmung stromabwärtigen Ende eine Strömungsabrisskante gebildet ist, an der sich die Blutkammer sprungartig, insbesondere in einem Winkel von mindestens 90° zum Verlauf des eingangsseitigen inkompressiblen Abschnitts der Blutkammer verbreitert. An dieser Stelle der Blutkammer kann somit eine Rückströmung des Blutes aus der Blutkammer hinaus unter Vermeidung von Totpunkten in der Strömung begünstigt werden. Totpunkte in der Strömung in der Blutkammer stellen ein erhebliches Risiko dar, da sie zu Thrombenbildung führen können. Durch die genannte strömungsgünstige Ausgestaltung der Blutkammer mithilfe der Strömungsabrisskante nach dem eingangsseitigen inkompressiblen Abschnitt der Blutkammer kann somit eine optimierte Blutströmung erreicht werden.

In einer weiteren bevorzugten Ausführungsform weist die Vorrichtung ein insbesondere implantierbares oder teil-implantierbares Reservoir zur Aufnahme einer größeren Menge des als Dialysats dienenden Hydraulikfluids als die Hydraulikkammer auf. Die bedeutet, dass in dem Reservoir eine größere Menge an Hydraulikfluid als in der Hydraulikkammer aufnehmbar ist. Das Hydraulikfluid, kann insbesondere durch die Pumpe vom Reservoir in die Hydraulikkammer und zurück gefördert werden. Bei einem Fördern von Hydraulikfluid vom Reservoir zur Hydraulikkammer erfolgt eine Verringerung der Konzentration von Abfallstoffen in dem in der Hydraulikkammer befindlichen Hydraulikfluid. Somit kann der Konzentrationsgradient zwischen dem Blut und dem Dialysat in der Hydraulikkammer über längere Zeit aufrecht erhalten werden, sodass ein Teilchenaustausch durch Diffusion über einen längeren Zeitraum möglich ist.

Es ist bevorzugt, dass das Reservoir eine Entleervorrichtung zum Abführen von Dialysat aus dem Reservoir aufweist. Hierbei kann es sich bspw. um eine Verbindung zu den harnführenden Wegen und/oder dem Darm des Patienten handeln, sodass die im Reservoir befindliche Flüssigkeit vom Patienten auf natürlichem Wege ausgeschieden werden kann. Bspw. kann die Verbindung zum Harnleiter eines Patienten mittels eines Steuerventils regelbar sein, wobei der Füllstand des Dialysats im Reservoir mittels eines Füllstandssensors bestimmbar ist. Das Steuerventil zum Herstellen einer fluidischen Verbindung zwischen dem Reservoir und den Harnwegen des Patienten kann bspw. mittels einer externen Steuereinheit oder mittels eines integrierten Reglers aktiviert werden. Es ist in diesem Zusammenhang bevorzugt, dass das Volumen der abgeführten Flüssigkeit gesteuert werden kann, sodass einem zu hohen Flüssigkeitsverlust beim Patienten vorgebeugt wird. Sofern das Reservoir mit den Harnwegen des Patienten verbunden ist, können die hierzu erforderlichen Nähte beschichtet werden, um die Bildung von Harnsteinen zu vermeiden.

Es ist weiterhin möglich, die im Dialysat innerhalb des Reservoirs befindlichen Abfallstoffe durch physikalische oder chemische Prozesse an größere Teilchen zu binden. Das Reservoir kann ein Filter aufweisen, durch das Wasser passieren kann, welches aber durch die an die größeren Teilchen gebundenen Abfallstoffe nicht passiert werden kann. Somit ist es möglich aus dem Reservoir, insbesondere kontinuierlich Wasser abzuführen, während die Abfallstoffe seltener, bspw. einmal wöchentlich entfernt werden können. Dies kann bspw. über ein Punktieren des Reservoirs an einem entsprechenden Port, der aus dem Stand der Technik bekannt ist, geschehen. Alternativ ist es möglich, die gesamte Flüssigkeit im Reservoir in regelmäßigen Abständen abzuführen, sodass der Konzentrationsgradient zwischen dem Blut und dem Dialysat langfristig aufrechterhalten werden kann.

In einer alternativen Ausführungsform weist die Dialysevorrichtung eine zweite Blutkammer, eine zweite Hydraulikkammer, eine zweite Fördermembran und eine zweite Filtermembran auf, die entsprechend der ersten Blutkammer, der ersten Hydraulikkammer, der ersten Fördermembran und der ersten Filtermembran zusammenwirken. Hierbei ist durch die Pumpe abwechselnd oder gleichzeitig Hydraulikfluid in die erste und zweite Hydraulikkammer und aus diesen hinaus förderbar, sodass ein gleichzeitiges oder abwechselndes Komprimieren und Expandieren der ersten und zweiten Blutkammer erfolgt. Bei dieser Ausführungsform der Vorrichtung sind die Porengrößen der ersten und zweiten Filtermembran unterschiedlich. Neben der Porengröße können auch weitere Membraneigenschaften, wie z.B. die Porendichte und/oder die Porenform variiert werden. Bspw. ist es möglich, dass die erste Filtermembran eine große Porengröße aufweist, sodass sie durch eine Vielzahl von Stoffen, die sich im Blut befinden, passiert werden kann. Durch Generieren eines Unterdrucks durch die Pumpe, in der ersten Hydraulikkammer wird ein Übergang dieser Stoffe von in der Blutkammer befindlichem Blut in das in der Hydraulikkammer befindliche Dialysat verursacht. Der Unterdruck in der ersten Hydraulikkammer kann dadurch erzeugt werden, dass die Pumpe Hydraulikfluid aus der ersten Hydraulikkammer herauspumpt. Durch das Herauspumpen dieses Hydraulikfluids aus der ersten Hydraulikkammer, das gleichzeitig als Dialysat dient, wird dieses der zweiten Hydraulikkammer zugeführt. In dieser wird durch die Pumpe ein Überdruck erzeugt, sodass die im Dialysat befindlichen Stoffe teilweise durch die zweite Filtermembran wieder in das in der zweiten Blutkammer befindliche Blut überführt werden können. Zum selektiven Überführen dieser Stoffe in das Blut kann die zweite Filtermembran z.B. kleinere Poren als die erste Filtermembran aufweisen, sodass diejenigen Stoffe, die nicht ins Blut gelangen sollen, im Dialysat zurückgehalten werden.

Weiterhin kann die erfindungsgemäße Vorrichtung mindestens einen Sensor zur Bestimmung der Systole im Kreislauf des Patienten aufweisen. Die Pumpe kann somit derart angesteuert werden, dass das Zuführen von Blut in die Blutkammer während der Systole erfolgt, sodass hierbei die Förderfunktion des natürlichen Blutkreislaufs ausgenutzt wird.

In einer weiteren bevorzugten Ausführungsform weist die Vorrichtung eine steife Druckplatte auf, die zwischen der Blutkammer und der Hydraulikkammer angeordnet ist und beim Befüllen der Hydraulikkammer mit Hydraulikfluid von der Hydraulikkammer in Richtung der Blutkammer verschiebbar ist, sodass durch die Druckplatte ein Komprimieren der Blutkammer erfolgt. Hierdurch kann ein gleichmäßigeres Komprimieren der Blutkammer erreicht werden.

Die Vorrichtung kann ferner eine Ladungserzeugungsvorrichtung zum Erzeugen einer elektrischen Ladung, z.B. in der Filtermembran oder an einer anderen Komponente aufweisen. Die elektrische Ladung sorgt dafür, dass Abfallprodukte im Hydraulikfluid abgestoßen werden, sodass sie daran gehindert werden in Richtung der Filtermembran zu strömen. Eine Rückströmung dieser Abfallprodukte in das Blut wird somit verhindert. Bspw. ist es möglich die Filtermembran oder eine andere Komponente mit einer elektrisch negativen Ladung zu beaufschlagen, sodass negativ geladene Moleküle im Blut, wie bspw. Eiweiße, abgestoßen werden. Hierdurch werden sie erstens daran gehindert, die Membran zu durchströmen und können sich weiterhin nicht in den Poren der Membran absetzen und diese somit verstopfen.

In einer weiteren bevorzugten Ausführungsform ist die an die Blutkammer angrenzende Hydraulikkammer eine erste Hydraulikkammer. Das Hydraulikfluid ist aus dieser ersten Hydraulikkammer durch eine Pumpe in eine zweite Hydraulikkammer und von dieser zurück förderbar. Hierbei kann es sich um dieselbe Pumpe, die bisher beschrieben wurde, handeln. Die zweite Hydraulikkammer grenzt an eine Sekundärdialysatkammer an und ist von dieser durch eine zweite Filtermembran getrennt, durch die Abfallprodukte von dem als Dialysat dienenden Hydraulikfluid an das Sekundärdialysat in der Sekundärdialysatkammer abführbar sind. In dieser Ausführungsform findet somit zunächst ein Übertritt von Abfallprodukten aus dem Blut durch die erste Filtermembran in Richtung des Hydraulikfluids oder Dialysats statt. Dieses stellt somit ein Primärdialysat dar. Das Primärdialysat kann auch Stoffe enthalten, die wieder zurück ins Blut gelangen sollen, z.B. Plasma. Die erste Filtermembran ist somit hinsichtlich ihrer Porengröße derart ausgebildet, dass derartige Substanzen die erste Filtermembran passieren können. Dagegen können diese Substanzen, die zweite Filtermembran nicht mehr passieren, da diese eine kleinere Porengröße aufweist. Die zweite Filtermembran kann entsprechend der ersten Filtermembran ebenfalls als Fördermembran dienen und das Sekundärdialysat in die Sekundärdialysatkammer hinein und aus dieser hinaus fördern. Es findet somit ein kontinuierlicher Austausch des Sekundärdialysats in der Sekundärdialysatkammer statt.

Zur Förderung des Hydraulikfluids können geeignete Minipumpen verwendet werden, die bspw. als Axial-, Diagonal-oder Verdrängerpumpen ausgebildet sind. Bspw. können Rollenzellen- oder Flügelzellen verwendet werden. Auch der Einsatz einer Kolbenpumpe oder eines Axialantriebs ist möglich. Die Pumpe kann unmittelbar im Gehäuse der Dialysevorrichtung oder aber im Reservoir angeordnet sein. Durch den Einsatz einer Pumpe zur Förderung des Bluts in die Blutkammer und aus diese hinaus kann gewährleistet werden, dass ein kontinuierlicher Austausch des in der Blutkammer befindlichen Blutes stattfindet, sodass eine effiziente Dialyse durchgeführt werden kann.

Zur Energieversorgung des Implantats kann keine kernlose, insbesondere flexible Empfangsspule vorgesehen sein, die induktiv mit einer extrakorporalen, insbesondere ebenfalls flexiblen Sendespule gekoppelt werden kann, sodass das Implantat durch induktive Kopplung mit Energie versorgt wird. Somit kann die Energieversorgung des Implantats durch transkutane Stromleitungen vermieden werden.

In einer weiteren bevorzugten Ausführungsform ist die Fördermembran derart ausgebildet, dass nur ein Teil des Blutvolumens aus der Blutkammer ausgeworfen werden kann. In dieser Ausführungsform kann die Blutkammer z.B. einen separaten Einlass und einen separaten Auslass aufweisen, die sich auf derselben Seite der Blutkammer befinden. Das Blut strömt somit durch den Einlass in die Blutkammer ein, wobei die Strömung des Bluts in der Blutkammer den Verlauf eines kleinen y aufweisen kann. Der Ein- und Auslass befinden sich hierbei an den beiden oberen Enden des kleinen y. Hierdurch kann eine besonders strömungsgünstige Ausgestaltung der Blutkammer erreicht werden. Es ist bevorzugt, dass in allen Ausführungsformen der Erfindung immer ein Restvolumen in der Blutkammer verbleibt, sodass die Blutkammer nie vollständig entleert wird. Hierdurch kann ein mechanisches Beschädigen eines in der Blutkammer immer vorhandenen Blutfilms verhindert werden.

Zur Bestimmung der Systole im Kreislauf des Patienten kann beispielsweise die Motorstromkurve der verwendeten Pumpe der Dialysevorrichtung betrachtet werden, sodass hier keine zusätzlichen Sensoren notwendig sind. Bei einer geringen Stromaufnahme der Pumpe kann bspw. von der Diastole ausgegangen werden während eine hohe Stromaufnahme der Pumpe auf die Systole hinweist.

Weiterhin ist es möglich, dass die Blutkammer derart ausgestaltet ist, dass sie von beiden Seiten komprimiert wird. Hierzu ist auf beiden Seiten der Blutkammer jeweils eine Hydraulikkammer angeordnet. Alternativ kann auch eine einzige Hydraulikkammer die Blutkammer umfassen und diese von beiden Seiten komprimieren. Somit ist es möglich, dass Blutvolumen gleichförmig aus der Blutkammer auszuwerfen.

Es ist ferner ein Verfahren beschrieben zum Betreiben einer portablen Dialysevorrichtung mit den folgenden Schritten:
Zunächst erfolgt ein kontinuierliches abwechselndes Befüllen und Entleeren der Hydraulikkammer mit einem Hydraulikfluid durch eine Pumpe.

Beim Befüllen der Hydraulikkammer wird eine wenigstens teilweise flexible Fördermembran in Richtung einer an die Hydraulikkammer angrenzenden Blutkammer bewegt, wodurch diese komprimiert wird. Beim Entleeren der Hydraulikkammer wird die Fördermembran von der Blutkammer wegbewegt, sodass diese expandiert.

Somit erfolgt ein kontinuierliches abwechselndes Komprimieren und Expandieren der Blutkammer durch die Fördermembran derart, dass ein kontinuierlicher ununterbrochener Austausch des in der Blutkammer befindlichen Bluts erfolgt.

Das Verfahren kann sämtliche Merkmale aufweisen, die im Zusammenhang mit der erfindungsgemäßen Vorrichtung beschrieben werden und umgekehrt.

Das Verfahren kann ferner die folgenden zusätzlichen Schritte aufweisen:
Die Systole und/oder Diastole im Kreislauf des Patienten kann durch mindestens einen Sensor detektiert werden. Zur Bestimmung der Systole oder Diastole kann anstelle eines Sensors auch der Motorstrom der Pumpe der erfindungsgemäßen Vorrichtung ausgewertet werden. Während der Diastole erfolgt ein Pumpen von Hydraulikfluid in die Hydraulikkammer. Alternativ oder zusätzlich kann während der Systole ein Pumpen von Hydraulikfluid aus der Hydraulikkammer hinaus erfolgen, sodass ein Befüllen der Blutkammer mit Blut während der Systole bzw. ein Entleeren der Blutkammer in den Kreislauf des Patienten während der Diastole erfolgt.

Weiterhin ist es möglich Abfallprodukte im Dialysat in der Hydraulikkammer, die nicht durch die Filtermembran zurück ins Blut gelangen sollen, in der Hydraulikkammer chemisch oder physikalisch an größere Partikel zu koppeln, die aufgrund ihrer Größe die Filtermembran nicht passieren können.

Es ist ferner die Verwendung eines Dialysats einer Dialysevorrichtung als Hydraulikflüssigkeit zum wechselweisen Komprimieren und Expandieren einer Blutkammer, unter Verwendung einer Fördermembran beschrieben, wobei die Blutkammer eine Filtermembran zum Abführen von Abfallstoffen aus dem Blut in das auf der anderen Seite der Filtermembran befindliche Dialysat aufweist.

Im Folgenden werden bevorzugte Ausführungsformen der Erfindung anhand von Figuren erläutert.

Es zeigen:
- Fig. 1: eine schematische Darstellung einer ersten Ausführungsform der erfindungsgemäßen Vorrichtung,
- Fig. 2: eine schematische Darstellung einer zweiten Ausführungsform der erfindungsgemäßen Vorrichtung,
- Fig. 3: eine schematische Darstellung einer dritten Ausführungsform der erfindungsgemäßen Vorrichtung,
- Fig. 4: eine schematische Darstellung einer vierten Ausführungsform der erfindungsgemäßen Vorrichtung,
- Fig. 5: verschiedene Ausführungsformen einer Fördermembran.

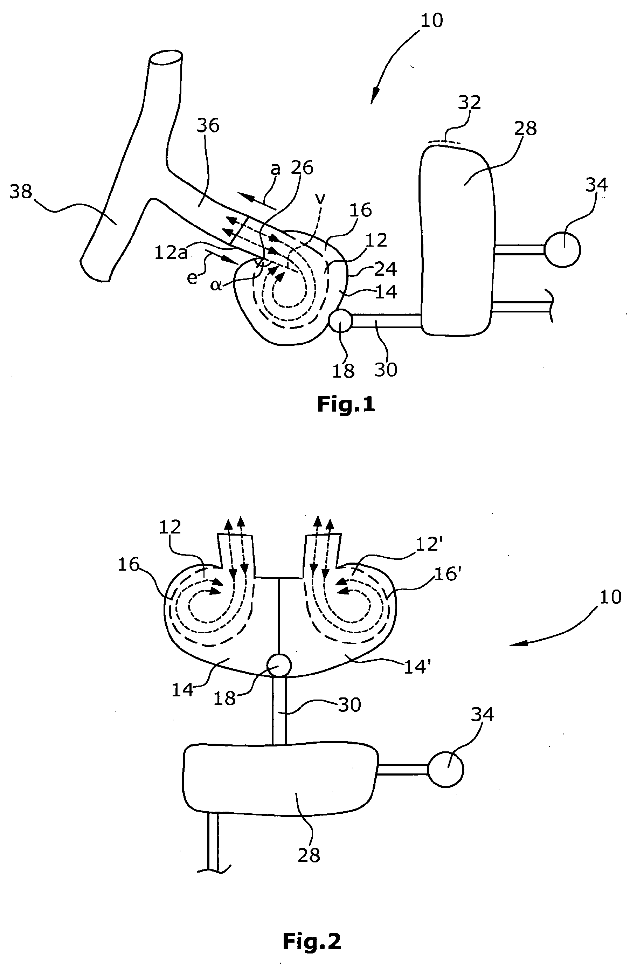

Wie in Fig. 1 dargestellt, weist die erfindungsgemäße portable Dialysevorrichtung 10 ein inkompressibles Gehäuse 24 auf, das strömungsgünstig ausgebildet ist. In dem Gehäuse 24 sind die Blutkammer 12 und die Hydraulikkammer 14 angeordnet, die durch die Filter- und Fördermembran 16 voneinander getrennt sind. Das Gehäuse 24 weist am Ende seines eingangsseitigen inkompressiblen Abschnitts 12a eine Strömungsabrisskante 26 auf. An dieser Stelle verbreitert sich das Gehäuse sprungartig in einem Winkel α, der größer als 90° ist. Die hierdurch erzeugte bzw. begünstigte Strömung des Blutes ist durch gestrichelte Linien in Fig. 1 dargestellt.

Das Bezugszeichen 30 bezeichnet in Fig. 1 eine Hydraulikleitung zum Zuführen von Hydraulikfluid in die Hydraulikkammer und aus dieser hinaus. Zwischen der Hydraulikkammer 14 und der Hydraulikleitung 30 ist die Pumpe 18 angeordnet. Am anderen Ende der Hydraulikleitung 30 ist das Reservoir 28 angeordnet, das ein Filter 32 zum Ausscheiden von Wasser und einen Port 34 zum Punktieren von Flüssigkeit aufweist.

Die Blutkammer 12 ist mittels einer Gefäßprothese 36 an ein Blutgefäß 38, z.B. die Aorta des Patienten, angeschlossen.

Durch den Einlass 12a strömt das Blut tangential an die innere Wand der Blutkammer 12, sodass eine günstige Strömung erzielt werden kann. In dieser Ausführungsform weist die Blutkammer 12 einen gemeinsamen Ein- und Auslass auf.

Das Volumen an Flüssigkeit, das die Filtermembran 16 passiert, kann bspw. bei 1 l bis 2 l pro 24 Stunden liegen.

Gemäß der in Fig. 2 dargestellten alternativen Ausführungsform weist die Dialysevorrichtung 10 eine erste und zweite Blutkammer 12,12', eine erste und zweite Hydraulikkammer 14,14' sowie eine erste und zweite Fördermembran 16,16' auf. Über die Pumpe 18 erfolgt ein abwechselndes oder gleichzeitiges Befüllen und Entleeren der Hydraulikkammern 14,14' mit Hydraulikfluid. In dieser Ausführungsform können die beiden Fördermembranen 16,16' unterschiedliche Porengrößen aufweisen.

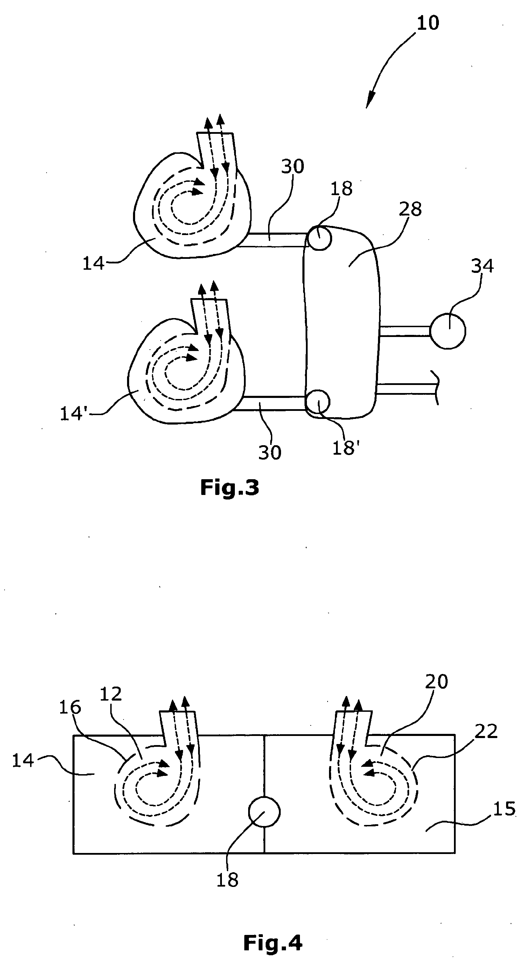

Fig. 3 ähnelt der Ausführungsform gemäß Fig. 2, wobei anstatt einer einzigen Pumpe zwei Pumpen 18,18' verwendet werden, die das Hydraulikfluid vom Reservoir 28 in die Hydraulikkammern 14,14' und zurück pumpen.

In der Ausführungsform gemäß Fig. 4 ist die Blutkammer 14 von einer Fördermembran 16 mit einer relativ großen Porengröße umgeben, durch die Stoffe hindurchtreten können, die wieder zurück in den Blutkreislauf gelangen sollen. Auch hier wird die Blutkammer 12 durch das Hydraulikfluid 14 komprimiert. Die Förderung des Hydraulikfluids erfolgt durch die Pumpe 18. Auf der rechten Seite der Vorrichtung gemäß Fig. 4 befindet sich die zweite Hydraulikkammer 15, die fluidisch von der Hydraulikkammer 14 getrennt sein kann. Die Pumpe 18 fördert somit abwechselnd das Hydraulikfluid von der ersten Hydraulikkammer 14 zur zweiten Hydraulikkammer 15 und zurück. Beim Pumpen des Hydraulikfluids in die zweite Hydraulikkammer 15 erfolgt ein Komprimieren der Sekundärdialysatkammer 20 durch die Fördermembran 22. Diese dient gleichzeitig als Filtermembran und weist eine geringere Porengröße als die Filtermembran 16 auf.

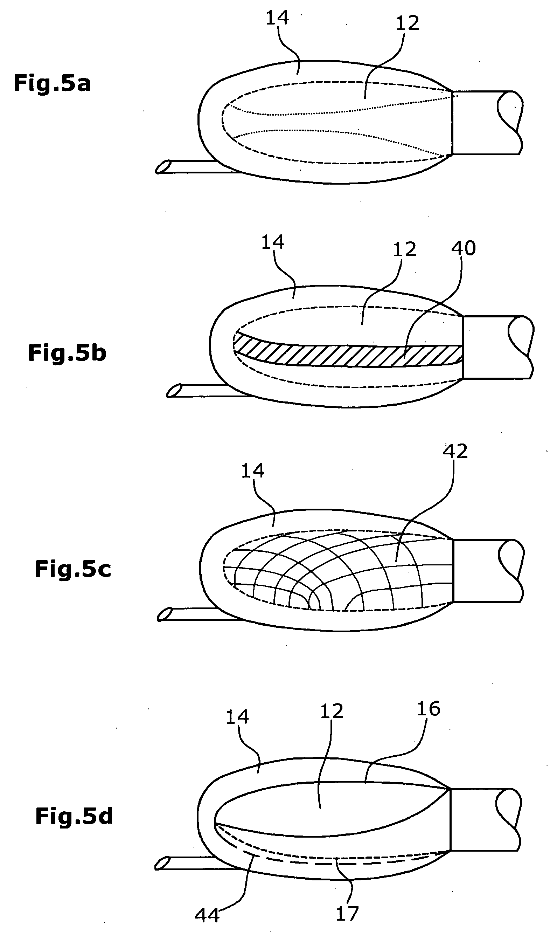

Wie in Fig. 5 gezeigt, können die Blutkammer 12 und die Hydraulikkammer 14 verschiedene Gestalten annehmen. Bspw. ist es möglich, die Blutkammer 12 von beiden aus zu komprimieren, indem die Hydraulikkammer 14 die Blutkammer 12 umgibt. Dies ist in den Figuren 5a bis 5d sichtbar. Der zusammengedrückte Zustand der Blutkammer 12 ist in Fig. 5a gestrichelt dargestellt.

Gemäß Fig. 5b ist ein äquatorförmiger Abschnitt 40 der Blutkammer 12 aus verstärktem Gewebe ausgebildet, sodass die Membran der Blutkammer 12 gestützt werden kann. Alternativ ist es möglich, eine Stützstruktur in Form eines Netzes 42 gemäß Fig. 5c vorzusehen. Das Netz 42 kann mit der Fördermembran 16 teilweise oder vollständig fest verbunden sein. Das Netz ist vorzugsweise flexibel ausgebildet. Gemäß Fig. 5d kann ein Teil der Fördermembran 16 durch ein starres Stützelement 44 gestützt sein, sodass der Pumpvorgang nur in dem flexiblen oberen Abschnitt der Fördermembran 16 stattfindet.

## Patentansprüche

1. Portable Dialysevorrichtung, die kontinuierlich im und/oder am Körper eines Patienten tragbar ist, mit
einer Blutkammer (12), in die Blut des Patienten aufnehmbar ist,
einer mit einem Hydraulikfluid befüllbaren Hydraulikkammer (14), die an die Blutkammer (12) angrenzt,
einer zwischen der Hydraulikkammer (14) und der Blutkammer (12) angeordneten wenigstens teilweise flexiblen Fördermembran (16), die bei Befüllung der Hydraulikkammer (14) mit Hydraulikfluid derart in Richtung der Blutkammer (12) bewegbar ist, dass ein Komprimieren der Blutkammer (12) und somit ein Auswurf des hierin befindlichen Blutes erfolgt,
einer Pumpe (18) zum steuerbaren Befüllen und/oder Entleeren des Hydraulikfluids in der Hydraulikkammer (14), sodass hierdurch Blut in die Blutkammer (12) und/oder aus dieser hinaus förderbar ist,
einer zwischen der Blutkammer (12) und der Hydraulikkammer (14) angeordneten Filtermembran (16,17), durch im Blut befindliche Abfallstoffe an das in der Hydraulikkammer (14) befindliche Hydraulikfluid abführbar sind, sodass das Hydraulikfluid gleichzeitig als Dialysat dient,
**dadurch gekennzeichnet, dass**
die Blutkammer (12) einen gemeinsamen Zu- und Abfluss aufweist, der nah an dem Blutgefäß (38) angeordnet ist, mit dem die Dialysevorrichtung (10) verbunden ist, sodass durch den ständigen Blutfluss im Blutgefäß (38) ein Weiterfördern des gereinigten Blutes, das von der Blutkammer (12) ausgeworfen wird, erfolgt, sodass beim nächsten Befüllen der Blutkammer (12) diese zum größten Teil mit frischem, noch nicht gereinigten Blut aus dem Blutgefäß (38) gefüllt wird,
die Blutkammer (12) derart ausgebildet ist, dass durch ihren Einlass (12a) das Blut tangential an die innere Wand der Blutkammer (12) strömt, sodass eine günstige Strömung erzielbar ist.

2. Portable Dialysevorrichtung nach Anspruch 1, **dadurch gekennzeichnet, dass** die Filtermembran (16) und die Fördermembran (16) dasselbe Bauteil (16) sind, sodass durch die Förder- und Filtermembran (16) gleichzeitig ein Komprimieren der Blutkammer (12) und ein Filtern des Bluts erfolgt.

3. Portable Dialysevorrichtung nach Anspruch 1, **dadurch gekennzeichnet, dass** die Filtermembran (17) und die Fördermembran (16) separate Bauteile sind und die Filtermembran (17) insbesondere unflexibel ist.

4. Portable Dialysevorrichtung nach einem der Ansprüche 1 bis 3, **dadurch gekennzeichnet, dass** die Drehrichtung der Pumpe (18) umkehrbar ist und insbesondere der durch die Pumpe (18) geförderte Volumenstrom des Hydraulikfluids regelbar ist, sodass durch die Pumpe (18) oder mit ihrer Unterstützung Blut in einem definierten Volumenstrom in die Blutkammer (12) und aus dieser hinaus förderbar ist.

5. Portable Dialysevorrichtung nach einem der Ansprüche 1 bis 4, **dadurch gekennzeichnet, dass** durch Regelung des durch die Pumpe (18) geförderten Volumenstroms des Hydraulikfluids der Druckgradient zwischen Blut und dem als Dialysat dienenden Hydraulikfluid zur Regelung der Blutreinigung durch Ultrafitration veränderbar ist, indem in der Hydraulikkammer (14) ein höherer oder niedrigerer Druck als in der Blutkammer (12) einstellbar ist.

6. Portable Dialysevorrichtung nach einem der Ansprüche 1 bis 5, **dadurch gekennzeichnet, dass** durch die Pumpe (18) ein kontinuierliches Fördern von Blut ohne Unterbrechung in die Blutkammer (12) und aus dieser hinaus erfolgt, wobei insbesondere der Volumenstrom des in die Blutkammer geförderten Bluts bei 0,4 l bis 2,5 I, insbesondere bei 0,8 l bis 1,5 l pro Minute bei erwachsenen Patienten und bei 50 ml bis 800 ml, insbesondere 100 ml bis 400 ml pro Minute bei Kindern liegt.

7. Portable Dialysevorrichtung nach einem der Ansprüche 1 bis 6, **dadurch gekennzeichnet, dass** die Blutkammer (12) und die Hydraulikkammer (14) in einem inkompressiblen Gehäuse (24) angeordnet sind und die Blutkammer (12) eingangsseitig einen inkompressiblen Abschnitt (12a) aufweist, an dessen bezüglich der Bluteinlassströmung (e) stromabwärtigen Ende eine Strömungsabrisskante (26) gebildet ist, an der sich die Blutkammer (12) sprungartig, insbesondere in einem Winkel (α) von mindestens 90° zum Verlauf (v) des eingangsseitigen inkompressiblen Abschnitts (12a) der Blutkammer (12) verbreitert, sodass an dieser Stelle eine Rückströmung des Blutes aus der Blutkammer (12) unter Vermeidung von Totpunkten in der Strömung begünstigt wird.

8. Portable Dialysevorrichtung nach einem der Ansprüche 1 bis 7, **gekennzeichnet durch** ein insbesondere implantierbares oder teil-implantierbares Reservoir (28) zur Aufnahme einer größeren Menge des als Dialysat dienenden Hydraulikfluids als die Hydraulikkammer (14), wobei insbesondere **durch** die Pumpe (18) das Hydraulikfluid vom Reservoir (28) in die Hydraulikkammer (14) und zurück förderbar ist, wobei bei einem Fördern von Hydraulikfluid vom Reservoir (28) in die Hydraulikkammer (14) eine Verringerung der Konzentration von Abfallstoffen in dem in der Hydraulikkammer (14) befindlichen Dialysat erfolgt,
wobei das Reservoir insbesondere eine Entleervorrichtung zum Abführen von Dialysat aus dem Reservoir aufweist, die insbesondere als Verbindung zu den harnführenden Wegen und/oder dem Darm des Patienten ausgebildet ist.

9. Portable Dialysevorrichtung nach einem der Ansprüche 1 bis 8, **gekennzeichnet durch** eine zweite Blutkammer (12'), eine zweite Hydraulikkammer (14'), eine zweite Fördermembran (16') und eine zweite Filtermembran (16'), die entsprechend der ersten Blutkammer (12), der ersten Hydraulikkammer (14), der ersten Fördermembran (16) und der ersten Filtermembran (16) zusammenwirken, wobei **durch** die Pumpe (18) abwechselnd oder gleichzeitig Hydraulikfluid in die erste und zweite Hydraulikkammer (14, 14') und aus diesen heraus förderbar ist, sodass ein gleichzeitiges oder abwechselndes Komprimieren und Expandieren der ersten und zweiten Blutkammer (12,12') erfolgt, wobei die Porengrößen der ersten und der zweiten Filtermembran (16,16') unterschiedlich sind.

10. Portable Dialysevorrichtung nach einem der Ansprüche 1 bis 9, **gekennzeichnet durch** mindestens einen Sensor zur Bestimmung der Systole im Kreislauf des Patienten derart, dass das Zuführen von Blut in die Blutkammer (12) während der Systole erfolgt.

11. Portable Dialysevorrichtung nach einem der Ansprüche 1 bis 10, **gekennzeichnet durch** eine steife Druckplatte zwischen der Blutkammer (12) und der Hydraulikkammer (14), die beim Befüllen der Hydraulikkammer (14) mit Hydraulikfluid von der Hydraulikkammer (14) in Richtung der Blutkammer (12) verschiebbar ist, sodass **durch** die Druckplatte ein Komprimieren der Blutkammer (12) erfolgt.

12. Portable Dialysevorrichtung nach einem der Ansprüche 1 bis 11, **gekennzeichnet durch** eine Ladungserzeugungsvorrichtung zum Erzeugen einer elektrischen Ladung an der Filtermembran (17), sodass Abfallprodukte im Hydraulikfluid von dieser elektrischen Ladung abgestoßen und daran gehindert werden in Richtung der Filtermembran (17) zu strömen.

13. Portable Dialysevorrichtung nach einem der Ansprüche 1 bis 12, **dadurch gekennzeichnet, dass** die an die Blutkammer (12) angrenzende Hydraulikkammer (14) eine erste Hydraulikkammer (14) ist und das Hydraulikfluid aus dieser ersten Hydraulikkammer (14) durch eine Pumpe (18) in eine zweite Hydraulikkammer (15) und von dieser zurück förderbar ist,
wobei die zweite Hydraulikkammer (15) an eine Sekundärdialysatkammer (20) angrenzt und von dieser durch eine zweite Filtermembran (22) getrennt ist, durch die Abfallprodukte von dem als Dialysat dienenden Hydraulikfluid an das Sekundärdialysat in der Sekundärdialysatkammer (20) abführbar sind.

## Claims

1. A portable dialysis device which is continuously portable in and/or on the body of a patient, comprising
a blood chamber (12) adapted to receive blood of the patient,
a hydraulic chamber (14) adapted to be filled with a hydraulic fluid, said hydraulic chamber being arranged adjacent to the blood chamber (12),
an least partially flexible conveying membrane (16), arranged between the hydraulic chamber (14) and the blood chamber (12), which, when the hydraulic chamber (14) is being filled with hydraulic fluid, is moveable in the direction of the blood chamber (12) in a manner causing compression of the blood chamber (12) and thus discharge of the blood contained in the blood chamber,
a pump (18) for controllable filling and/or discharging the hydraulic fluid in the hydraulic chamber (14) for thus conveying blood into the blood chamber (12) and/or out therefrom,
a filtering membrane (16,17), arranged between the blood chamber (12) and the hydraulic chamber (14), through which waste substances contained in the blood can be discharged to the hydraulic fluid arranged in the hydraulic chamber (14), so that the hydraulic fluid at the same time is functional as a dialysate,
**characterized in that**
the blood chamber (12) comprises a combined inlet and outlet arranged close to the blood vessel (38) and having the dialysis device (10) connected to it so that, due to the steady blood flow in the blood vessel (38), the purified blood ejected from the blood chamber (12) is conveyed further onwards so that, during the next filling of the blood chamber (12), the blood chamber will be filled for the largest part by fresh, not yet purified blood from the blood vessel (38),
the blood chamber (12) is designed in such a manner that the blood will flow tangentially through its inlet (12a) to the inner wall of the blood chamber (12) so that a favorable flow can be obtained.

2. The portable dialysis device according to claim 1, **characterized in that** the filtering membrane (16) and the conveying membrane (16) are the same component (16) so that the filtering and conveying membrane (16) is operative to compress the blood chamber (12) and to filter the blood at the same time.

3. The portable dialysis device according to claim 1, **characterized in that** the filtering membrane (17) and the conveying membrane (16) are separate components and the filtering membrane (17) is preferably inflexible.

4. The portable dialysis device according to any one of claims 1 to 3, **characterized in that** the rotational direction of the pump (18) is reversible and, preferably, the volume flow of the hydraulic fluid conveyed by the pump (18) is controllable so that, by the pump (18) or assisted by it, blood is conveyable in a defined volume flow into and out of the blood chamber (12).

5. The portable dialysis device according to any one of claims 1 to 4, **characterized in that**, by control of the volume flow of the hydraulic fluid conveyed by the pump (18), the pressure gradient between blood and the hydraulic fluid functional as a dialysate is controllable for control of the blood purification by ultrafiltration **in that**, in the hydraulic chamber (14), there is settable a higher or lower pressure than in the blood chamber (12).

6. The portable dialysis device according to any one of claims 1 to 5, **characterized in that**, by means of the pump (18), a continuous conveyance of blood into and out of the blood chamber (12) is performed without interruption, wherein, preferably, the volume flow of the blood conveyed into the blood chamber is 0.4 l to 2.5 l and more preferably 0.8 l to 1.5 l per minute for adults and 50 ml to 800 ml and more preferably 100 ml to 400 ml per minute for children.

7. The portable dialysis device according to any one of claims 1 to 6, **characterized in that** the blood chamber (12) and the hydraulic chamber (14) are arranged in an incompressible housing (24) and that the blood chamber (12) at its input side comprises an incompressible section (12a) which on its downstream end relative to the blood intake flow (e) is provided with a flow cut-off edge (26) where the blood chamber (12) is abruptly widened, preferably at an angle (α) of at least 90° relative to the development (v) of said incompressible section (12a) arranged at the input side of the blood chamber (12), so that, on this site, a backflow of blood from the blood chamber (12) is enhanced while avoiding dead points in the flow.

8. The portable dialysis device according to any one of claims 1 to 7, **characterized by** a preferably implantable or partly implantable reservoir (28) for receiving a larger quantity of the hydraulic fluid functional as a dialysate than the hydraulic chamber (14), wherein, preferably, by means of the pump (18), the hydraulic fluid is conveyable from the reservoir (28) into the hydraulic chamber (14) and back, wherein, during conveyance of hydraulic fluid from the reservoir (28) into the hydraulic chamber (14), the concentration of waste substances in the dialysate contained in the hydraulic chamber (14) is reduced,
wherein the reservoir preferably comprises a vacating device for discharge of dialysate from the reservoir, said vacating device being preferably formed as a connection to the urine-conducting ducts and/or to the intestines of the patient.

9. The portable dialysis device according to any one of claims 1 to 8, **characterized by** a second blood chamber (12'), a second hydraulic chamber (14'), a second conveying membrane (16') and a second filtering membrane (16') which are operative to cooperate in a manner corresponding to the first blood chamber (12), the first hydraulic chamber (14), the first conveying membrane (16) and the first filtering membrane (16), wherein, by the pump (18), hydraulic fluid is conveyable alternately or simultaneously into and out from the first and second hydraulic chambers (14,14') so that the first and second blood chambers (12,12') are simultaneously or alternately compressed and expanded, wherein the pore sizes of the first and second filtering membranes (16,16') are different.

10. The portable dialysis device according to any one of claims 1 to 9, **characterized by** at least one sensor for determining the systole in the blood circulation of the patient in such a manner that the supply of blood into the blood chamber (12) is performed during the systole.

11. The portable dialysis device according to any one of claims 1 to 10, **characterized by** a stiff pressure plate, arranged between the blood chamber (12) and the hydraulic chamber (14), which, while the hydraulic chamber (14) is being filled with hydraulic fluid, is displaceable from the hydraulic chamber (14) in the direction of the blood chamber (12) so that the blood chamber (12) is compressed by the pressure plate.

12. The portable dialysis device according to any one of claims 1 to 11, **characterized by** a charge generating device for generating an electric charge at the filtering membrane (17), so that waste products in the hydraulic fluid are repelled by said electric charge and are hindered from flowing in the direction of the filtering membrane (17).

13. The portable dialysis device according to any one of claims 1 to 12, **characterized in that** the hydraulic chamber (14) adjacent to the blood chamber (12) is a first hydraulic chamber (14) and the hydraulic fluid is conveyable from said first hydraulic chamber (14) via a pump (18) into a second hydraulic chamber (15) and back from the latter,
wherein said second hydraulic chamber (15) is arranged adjacent to a secondary dialysate chamber (20) and is separated therefrom by a second filtering membrane (22) which is operative to discharge waste products from the hydraulic fluid functional as a dialysate to the secondary dialysate in the secondary dialysate chamber (20).

## Revendications

1. Dispositif de dialyse portable, qui peut être porté de façon continue dans et/ou sur le corps d'un patient, avec
une chambre pour le sang (12) dans laquelle du sang du patient peut être reçu,
une chambre hydraulique (14) pouvant être remplie d'un fluide hydraulique, qui est adjacente à la chambre pour le sang (12),
une membrane de transport (16) au moins partiellement flexible, disposée entre la chambre hydraulique (14) et la chambre pour le sang (12), qui, lors du remplissage de la chambre hydraulique (14) avec du fluide hydraulique, peut être déplacée en direction de la chambre pour le sang (12) de telle sorte qu'ils se produit une compression de la chambre pour le sang (12) et donc une expulsion du sang qui s'y trouve,
une pompe (18) pour le remplissage et/ou le vidage de manière contrôlée du fluide hydraulique de la chambre hydraulique (14) de telle sorte que le sang peut ainsi être transporté dans la chambre pour le sang (12) et/ou évacué de celle-ci,
une membrane filtrante (16, 17), disposée entre la chambre pour le sang (12) et la chambre hydraulique (14), par laquelle des déchets présents dans le sang peuvent être évacués vers le fluide hydraulique présent dans la chambre hydraulique (14) de telle sorte que le fluide hydraulique sert en même temps de dialysat,
**caractérisé en ce que**
la chambre pour le sang (12) présente une admission et une sortie qui sont disposées à proximité du vaisseau sanguin (38) auquel est raccordé le dispositif de dialyse (10) de telle sorte que le flux sanguin constant dans le vaisseau sanguin (38) produit une continuation du transport du sang purifié qui est éjecté de la chambre pour le sang (12) de telle sorte que, lors du remplissage suivant de la chambre pour le sang (12), celle-ci est remplie en majeure partie avec du sang frais, non encore purifié, en provenance du vaisseau sanguin (38),
la chambre pour le sang (12) est constituée de telle sorte que, à travers son entrée (12a), le sang s'écoule tangentiellement à là paroi intérieure de la chambre pour le sang (12) de telle sorte qu'il est possible d'obtenir un écoulement favorable.

2. Dispositif de dialyse portable selon la revendication 1, **caractérisé en ce que** la membrane filtrante (16) et la membrane de transport (16) sont le même composant (16) de telle sorte qu'une compression de la chambre pour le sang (12) et une filtration du sang sont effectuées simultanément par la membrane de transport et filtrante (16).

3. Dispositif de dialyse portable selon la revendication 1, **caractérisé en ce que** la membrane filtrante (17) et la membrane de transport (16) sont des composants séparés et **en ce que** la membrane filtrante (17) est en particulier non flexible.

4. Dispositif de dialyse portable selon l'une des revendications 1 à 3, **caractérisé en ce que** le sens de rotation de la pompe (18) peut être inversé et **en ce que**, en particulier, le flux volumique du fluide hydraulique transporté par la pompe (18) peut être régulé de telle sorte que, par la pompe (18) ou avec son aide, le sang peut être transporté dans la chambre pour le sang (12) et/ou évacué de celle-ci à raison d'un flux volumique défini.

5. Dispositif de dialyse portable selon l'une des revendications 1 à 4, **caractérisé en ce que**, du fait de la régulation du flux volumique du fluide hydraulique transporté par la pompe (18), le gradient de pression entre le sang et le fluide hydraulique servant de dialysat peut être modifié pour la régulation de la purification du sang par ultrafiltration par le fait qu'une pression plus élevée ou plus faible que dans la chambre pour le sang (12) peut être réglée dans la chambre hydraulique (14).

6. Dispositif de dialyse portable selon l'une des revendications 1 à 5, **caractérisé en ce qu'**un transport continu de sang sans interruption vers la chambre pour le sang (12) et hors de celle-ci est effectué par la pompe (18), en particulier le flux volumique du sang transporté dans la chambre pour le sang étant compris entre 0,4 1 et 2,5 l, en particulier entre 0,8 1 et 1,5 1 par minute chez des patients adultes, et entre 50 ml et 800 ml, en particulier entre 100 ml et 400 ml par minute chez des enfants.

7. Dispositif de dialyse portable selon l'une des revendications 1 à 6, **caractérisé en ce que** la chambre pour le sang (12) et la chambre hydraulique (14) sont disposées dans un boîtier (24) incompressible, et la chambre pour le sang (12) présente, côté entrée, un tronçon (12a) incompressible à l'extrémité duquel, en aval par rapport à l'écoulement d'entrée du sang (e), il est formé une arête de décrochage d'écoulement (26) au niveau de laquelle la chambre pour le sang (12) s'élargit brusquement, en particulier en formant un angle (α) d'au moins 90° par rapport au tracé (v) du tronçon (12a) incompressible, côté entrée, de la chambre pour le sang (12) de telle sorte qu'à cet endroit un reflux du sang à partir de la chambre pour le sang (12) est favorisé en évitant des points morts dans l'écoulement.

8. Dispositif de dialyse portable selon l'une des revendications 1 à 7, **caractérisé par** un réservoir (28) en particulier implantable ou partiellement implantable, destiné à recevoir une quantité de fluide hydraulique servant de dialysat plus importante que la chambre hydraulique (14), le fluide hydraulique pouvant en particulier être transporté par la pompe (18) à partir du réservoir (28) vers la chambre hydraulique (14) et inversement, une réduction de la concentration de déchets dans le dialysat situé dans la chambre hydraulique (14) se produisant lors d'un transport du fluide hydraulique à partir du réservoir (28) vers la chambre hydraulique (14),
le réservoir présentant en particulier un dispositif de vidage pour l'évacuation du dialysat hors du réservoir qui est constitué en particulier en tant que raccordement aux voies urinaires et/ou à l'intestin du patient.

9. Dispositif de dialyse portable selon l'une des revendications 1 à 8, **caractérisé par** une deuxième chambre pour le sang (12'), une deuxième chambre hydraulique (14'), une deuxième membrane de transport (16') et une deuxième membrane filtrante (16') qui coopèrent conformément à la première chambre pour le sang (12), à la première chambre hydraulique (14), à la première membrane de transport (16) et à la première membrane filtrante (16), du fluide hydraulique pouvant être transporté dans la première et la deuxième chambre hydraulique (14, 14') et évacué de celles-ci par la pompe (18), de façon alternée ou simultanément, de telle sorte qu'il se produit simultanément ou de façon alternée une compression et une expansion de la première et de la deuxième chambre pour le sang (12, 12'), les grandeurs de pores de la première et de la deuxième membrane filtrante (16, 16') étant différentes.

10. Dispositif de dialyse portable selon l'une des revendications 1 à 9, **caractérisé par** au moins un capteur pour la détermination de la systole dans la circulation du patient de telle sorte que
l'acheminement de sang dans la chambre pour le sang (12) se produit pendant la systole.

11. Dispositif de dialyse portable selon l'une des revendications 1 à 10, **caractérisé par** une plaque de pression rigide entre la chambre pour le sang (12) et la chambre hydraulique (14) qui peut, lors du remplissage de la chambre hydraulique (14) avec du fluide hydraulique, être déplacée à partir de la chambre hydraulique (14) en direction de la chambre pour le sang (12) de telle sorte que la plaque de pression produit une compression de la chambre pour le sang (12).

12. Dispositif de dialyse portable selon l'une des revendications 1 à 11, **caractérisé par** un dispositif de production de charge pour la production d'une charge électrique sur la membrane filtrante (17) de telle sorte que des produits de rebut dans le fluide hydraulique sont repoussés par cette charge électrique et sont empêchés de s'écouler en direction de la membrane filtrante (17).

13. Dispositif de dialyse portable selon l'une des revendications 1 à 12, **caractérisé en ce que** la chambre hydraulique (14) jouxtant la chambre pour le sang (12) est une première chambre hydraulique (14), et le fluide hydraulique peut être transporté hors de cette première chambre hydraulique (14) par une pompe (18) vers une deuxième chambre hydraulique (15) et inversement,
la deuxième chambre hydraulique (15) jouxtant une chambre de dialysat secondaire (20) et étant séparée de celle-ci par une deuxième membrane filtrante (22) par laquelle des produits de rebut du fluide hydraulique servant de dialysat peuvent être évacués vers le dialysat secondaire dans la chambre de dialysat secondaire (20).
